# EUROPEAN PATENT APPLICATION

(11) **EP 4 649 952 A1**
(43) Date of publication of application: **19.11.2025**
(21) Application number: 24200831.6
(22) Date of filing: 17.09.2024
(51) Int. Cl.: A61K 35/17, A61K 39/00, A61P 35/00, C07K 14/725

(54) **KRAS Q16H SPECIFIC T CELL RECEPTORS AND USES THEREOF**

(30) Priority: 14.05.2024 EP 24175796
(71) Applicant: HS Diagnomics GmbH, 12163 Berlin (DE)
(72) Inventor: The designation of the inventor has not yet been filed
(74) Representative: Schulz Junghans Patentanwälte PartGmbB

(57) **Abstract**

The invention relates to isolated T cell receptors specific for Q61H mutations in the KRAS cancer oncogene, and engineered cells expressing such T cell receptors. Further aspects of the invention relate to therapeutic use of T cell receptors and cells according to the invention in treating cancer, as well as methods of identifying patients who will derive a therapeutic benefit from these treatments.

## Description

### Field

The present invention relates to therapeutic T cell products expressing a T cell receptor specific for a Q61H mutation in members of the RAS family of proteins.

This application claims the right of priority of European Patent Application EP24175796.2 filed 14 May 2024, which is incorporated by reference herein.

### Background

Adoptive cell therapy (ACT) with genetically engineered T cells expressing chimeric antigen receptors (CAR-T cells) specific for lineage antigens has shown promise and has been approved for treating several hematologic malignancies (1,2) . However, expanding CAR-T therapies to solid tumors has faced hurdles, such as a lack of tumor-specific cell surface antigens (3). Unlike CARs, TCRs can target antigens from any part of a tumor cell, including intracellular tumor-associated and tumor-specific antigens (TAA, TSA) (4-6). Recent progress in TIL therapies supports this concept (7). TCR recognition depends on peptide presentation by HLA molecules, a multigenic and polymorphic system that presents a vast variety of immunogenic peptides. However, this means a therapeutic TCR can only be applied to patients with the appropriate HLA allele and specific TAA/TSA combination. Therefore, most clinical TCR-T trials have focused on peptides from common TAAs presented by the prevalent HLA-A02:01(8). Despite these achievements, such tumor-reactive TCR-based therapies remain inaccessible to most patients due to the challenges of targeting TAAs, including the risk of on-target/off-tumor reactivity because of their non-tumor-specific expression. Expression of TAAs in normal tissues has resulted in severe autoimmune adverse events in TCR-T clinical trials (9,10), including fatal events involving affinity-optimized TCRs against the TAA MAGE-A3 (11).

Such toxicities can be avoided by utilizing natural TCRs that target TSAs, which include all types of somatic non-synonymous mutations in canonical proteins and aberrantly transcribed and translated gene products, collectively known as neoantigens. Neoantigens play a crucial role in the effects of immune checkpoint inhibition (ICI) and TIL therapy. Nevertheless, only a small fraction of mutated gene products have been found to be immunogenic. Moreover, most neoepitopes are unique to individual tumors, posing challenges for therapeutic application due to the need for personalization, heterogeneity in neoantigen expression across tumors and metastases, and the time and effort required to identify effective TCR-neoepitope combinations (12). Still, personalized TCR-T cell therapy approaches targeting individual neoepitopes in patients with refractory solid cancers are being developed. Neoepitopes derived from recurrent mutations in oncogenic driver genes are considered optimal therapeutic targets due to their roles in tumorigenesis and progression and their clonal and stable expression across lesions. However, few immunogenic driver mutations have been identified so far. While naturally occurring T cells specific for recurrent neoantigens have been rarely reported in patients, the therapeutic activity of TCRs targeting this type of antigen has been demonstrated in clinical settings. For example, TCR-T cells targeting a KRAS G12D-peptide presented by HLA-C*08:02 induced partial responses in patients with metastatic colorectal and pancreatic cancer, while TCR-T cells specific for TP53 R175H showed clinical activity in a patient with metastatic breast cancer (13-15).

Based on the above-mentioned state of the art, the objective of the present invention is to provide means and methods to obtain effective and safe T cell-based cell therapy agents specific for immunogenic tumor neoantigens that recur in diverse cancer patients. This objective is attained by the subject-matter of the independent claims of the present specification, with further advantageous embodiments described in the dependent claims, examples, figures and general description of this specification.

### Summary of the Invention

The TCRs described herein were identified via an antigen-agnostic approach for identifying tumor-specific T-cell clonotypes by comparing high-throughput TCR-repertoire profiles of tumor and adjacent normal tissue-infiltrating T-cell clonotypes from surgical specimens. A clonal family of identical, or nearly identical tumor-specific TCRs were identified in non-small cell lung cancer (NSCLC) patients. Engineered TCR-T cells bearing these TCRs recognized autologous tumor cells and HLA-matched NSCLC cell lines. Screening for recognition of expressed non-synonymous neoepitopes and overexpressed TAA candidates revealed that the selected TCRs specifically recognized mutant (m)KRAS Q61H-peptide 55-64 (ILDTAGHEEY, highlighted amino acid substitution) presented by HLA-A*01:01, present in cancer cells from various tissues.

The potential therapeutic benefit of the selected TCRs is supported by a) functional characterization of TCR-T cells genetically engineered with the TCRs, b) phenotypic characterization of the original TIL clonotypes, c) the presence of these clonotypes in tumor relapse acquired more than 30 months after primary tumor surgery, and d) the discovery of identical or highly homologous TCRs in six out of 29 archival (FFPE) tumor samples with confirmed Q61H mutations. The mKRAS-reactive TCRs described here can be used as TCRs for adoptive cell therapy in HLA-A*01:01-positive patients with m(K/H/N)RAS Q61H-positive tumors.

A first aspect of the invention relates to an isolated T cell receptor (TCR) comprising an alpha T cell receptor (TCRα) polypeptide comprising a complementarity determining region 3 alpha (CDR3α) sequence and beta T cell receptor (TCRβ) polypeptide comprising a complementarity determining region 3 alpha (CDR3β) sequence. According to this invention, the CDR3α sequence of the TCR's TCRα chain comprises:
CALSEA(XXX)¹G(XX)²LX³F, wherein (XXX)¹ is NQA, GSS, or KAA, (XX)² is TA, SA, or NK, and X³ is I or T (SEQ ID NO 022).

In addition, the β sequence of the TCRβ chain of the TCR comprises:
CASSR(XX)¹PP(XXXXX)²F, wherein (XX)¹ is KV, RW, RR or KL, and (XXXXX)² is VYGYT, VYGDT, TDTQY, or TYGYT (SEQ ID NO 023).

In some embodiments, the TCR comprises a TCRα and/or TCRβ chain with a variant CDR3 having one, or two amino acid substitutions compared to SEQ ID NO 022, and/or SEQ ID NO 023, respectively.

Further aspects of the invention relate to a nucleic acid encoding components of the isolated TCR according to the previous aspect of the invention, or a nucleic acid expression vector encoding same.

Another important aspect of the invention is an engineered T cell expressing a TCR as specified according to the first aspect of the invention, which is shown to confer strong effector cell reactivity to tumor cells presenting a peptide derived from a RAS family protein having a Q61H mutation, particularly the peptide SEQ ID NO 021.

Further aspects of the invention relate to such isolated TCR, and/or engineered T cells expressing these TCR, and pharmaceutical compositions comprising protein, or cellular therapeutic agents comprising these elements for use in treating cancer patients whose tumor has a Q61H mutation.

Further aspects of the invention relate to methods of selecting cancer patients who will receive a therapeutic benefit from the cancer treatments according to the aspects of the invention provided above, or methods of monitoring the T cell responses of such patients to track treatment efficacy or receptivity.

### Terms and definitions

### General

For purposes of interpreting this specification, the following definitions will apply and whenever appropriate, terms used in the singular will also include the plural and vice versa. In the event that any definition set forth below conflicts with any document incorporated herein by reference, the definition set forth shall control.

The terms "comprising", "having", "containing", and "including", and other similar forms, and grammatical equivalents thereof, as used herein, are intended to be equivalent in meaning and to be open-ended in that an item or items following any one of these words is not meant to be an exhaustive listing of such item or items, or meant to be limited to only the listed item or items. For example, an article "comprising" components A, B, and C can consist of (i.e., contain only) components A, B, and C, or can contain not only components A, B, and C but also one or more other components. As such, it is intended and understood that "comprises" and similar forms thereof, and grammatical equivalents thereof, include disclosure of embodiments of "consisting essentially of" or "consisting of."

Where a range of values is provided, it is understood that each intervening value, to the tenth of the unit of the lower limit, unless the context clearly dictates otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.

Reference to "about" a value or parameter herein includes (and describes) variations that are directed to that value or parameter per se. For example, description referring to "about X" includes description of "X."

As used herein, including in the appended claims, the singular forms "a", "or" and "the" include plural referents unless the context clearly dictates otherwise.

"And/or" where used herein is to be taken as specific recitation of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

Unless defined otherwise, all technical and scientific terms used herein have the same meaning as commonly understood by one of ordinary skill in the art (e.g., in cell culture, molecular genetics, nucleic acid chemistry, hybridization techniques and biochemistry, organic synthesis). Standard techniques are used for molecular, genetic, and biochemical methods (see generally, Sambrook et al., Molecular Cloning: A Laboratory Manual, 4th ed. (2012) Cold Spring Harbor Laboratory Press, Cold Spring Harbor, N.Y. and Ausubel et al., Short Protocols in Molecular Biology (2002) 5th Ed, John Wiley & Sons, Inc.) and chemical methods.

Any patent document cited herein shall be deemed incorporated by reference herein in its entirety.

### T cell biology

The term "TCR" in the context of the present specification refers to a T cell receptor.

A TCR is formed from a dimer of a TCR alpha (TCRα) polypeptide, and a TCR beta (TCRβ) polypeptide, sometimes referred to as the TCRα and TCRβ TCR chains. TCR chains are formed by recombination of multiple genes to form a polypeptide comprising both a variable region, important for antigen recognition, and a constant region contributing to functional protein signaling. The extracellular portion of the TCR comprises hypervariable complementarity determining regions (CDR) surrounded by a less variable framework domains. The variable domains of the TCR are also known as a Vβ, or Vα (or Vγ or Vδ in the case of a gamma delta T cell) segments. The pair of complementary hypervariable CDR3 regions in a TCR heterodimer are a unique identifier of an individual T cell clone, conferring specificity to a specific antigenic molecule presented in the context of an MHC protein on the surface of an antigen presenting cell. The constant region of the TCR comprises a connecting peptide, a transmembrane domain, and an intracellular domain.

The various TCR domains have a conserved structure, which permit numbering of the amino acid residues, and comparisons of TCR chain amino acid sequences, within and among sequences, according to standard numbering sequences such as the Kabat, or IMGT systems. Identification and numbering of TCR chain domain sequences may be carried out for example, by entering the full amino acid, (or nucleic acid sequence encoding said amino acid sequence) into an online tool such as that provided by the international ImMunoGeneTics (IMGT) information system. Alternatively, CDR3, or variable domain sequences may be annotated within a full TCR chain sequence using software packages such as MiTCR, MiXCR, or Immcantation - Change-O (Bolotin et al. 2013, Nat. Meth. 10:813; Bolotin et al. 2015, Nat. Meth. 12:380; Gupta et al. 2015, Bioinformatics 31:3356). Such tools and software packages known in the art can annotate the variable domain encoding the first framework region, the CDR1, the second framework region, the CDR2, the third framework region, and the junction comprising the CDR3 which indicates the C-terminal end of the variable domain.

The term "CDR3" in the context of the present specification refers to the hypervariable complementarity determining region 3. The size of CDR3 is particularly characterized by the total number of amino acids (AA) and respective nucleotides from the conserved cysteine in the Vβ, or Vα or Vγ or Vδ segment to the position of the conserved phenylalanine in the Jβ or Jα, Jγ or Jδ segment.

The TCR amino acid sequences provided herein are defined by a pair of CDR3 sequences, or by full variable domain sequences comprising the amino acids essential for antigen recognition in the context of specific HLA. The skilled artisan will recognize a functional TCR receptor polypeptide expressed from a transgene using such a variable domain will likely further comprise at the N-terminal end a leader signal (cleaved after synthesis), followed by the variable domain (V), and at the C-terminal end will likely further comprise a constant domain.

The term *HLA* in the context of the present specification relates to the products on the major histocompatibility complex (MHC), in humans known as the human leukocyte antigen (HLA) located on chromosome 6, having the biological role of presenting processed peptide antigens to specifically reactive T cell receptors on T cells. Polymorphic expression of *HLA* among humans requires molecular HLA typing of patients to be routinely performed in the clinic, for example in transplantation procedures, to identify the specific HLA class I and class II alleles expressed as proteins, inherited in a Mendelian fashion from each parent.

The term *HLA allele* in the context of the present specification relates to the gene, or gene product of the HLA class I gene family mediating antigen presentation to T cells. *HLA class I alleles* HLA-A, HLA-B and HLA-C genes encode glycosylated heavy chains which associate non covalently with an invariant beta-2-microglobulin chain to provide a membrane bound structure forming a peptide-binding groove between two alpha helices and a beta-pleated floor, to provide a binding site for processed peptide antigen from within the cell. The HLA allele type of a cancer patient may be assessed by clinical HLA-typing assays known in the art, particularly high-resolution HLA gene sequencing of a DNA sample obtained from the patient.

### Sequences

Sequences similar or homologous (e.g., at least about 70% sequence identity) to the sequences disclosed herein are also part of the invention. In some embodiments, the sequence identity at the amino acid level can be about 80%, 85%, 90%, 91%, 92%, 93%, 94%, 95%, 96%, 97%, 98%, 99% or higher. At the nucleic acid level, the sequence identity can be about 70%, 75%, 80%, 85%, 90%, 91%, 92%, 93%, 94%, 95%, 96%, 97%, 98%, 99% or higher. Alternatively, substantial identity exists when the nucleic acid segments will hybridize under selective hybridization conditions (e.g., very high stringency hybridization conditions), to the complement of the strand. The nucleic acids may be present in whole cells, in a cell lysate, or in a partially purified or substantially pure form.

In the context of the present specification, the terms *sequence identity* and *percentage of sequence identity* refer to a single quantitative parameter representing the result of a sequence comparison determined by comparing two aligned sequences position by position. Methods for alignment of sequences for comparison are well-known in the art. Alignment of sequences for comparison may be conducted by the local homology algorithm of Smith and Waterman, Adv. Appl. Math. 2:482 (1981), by the global alignment algorithm of Needleman and Wunsch, J. Mol. Biol. 48:443 (1970), by the search for similarity method of Pearson and Lipman, Proc. Nat. Acad. Sci. 85:2444 (1988) or by computerized implementations of these algorithms, including, but not limited to: CLUSTAL, GAP, BESTFIT, BLAST, FASTA and TFASTA.

Software for performing BLAST analyses is publicly available, e.g., through the National Center for Biotechnology-Information (http://blast.ncbi.nim.nih.gov/).

One example for comparison of amino acid sequences is the BLASTP or TBLASTX algorithm that uses very specific settings to take into account that the CDR3 regions of TCRs are very small in length (9-14 Aa). The precise parameters have to be adjusted with respect to e.g. size of the BLAST database, a typical setting is: E-value of 100; Word size: 2; minimum percentage similarity 60%; Matrix: BLOSUM62; default for the remaining parameters. Unless stated otherwise, sequence identity values provided herein refer to the value obtained using the BLAST suite of programs (Altschul et al., J. Mol. Biol. 215:403-410 (1990)) using the above identified default parameters for protein and nucleic acid comparison, respectively.

Reference to identical sequences without specification of a percentage value implies 100% identical sequences (i.e. the same sequence).

Particular embodiments make use of the sequences as disclosed herein (i.e. 100% identical).

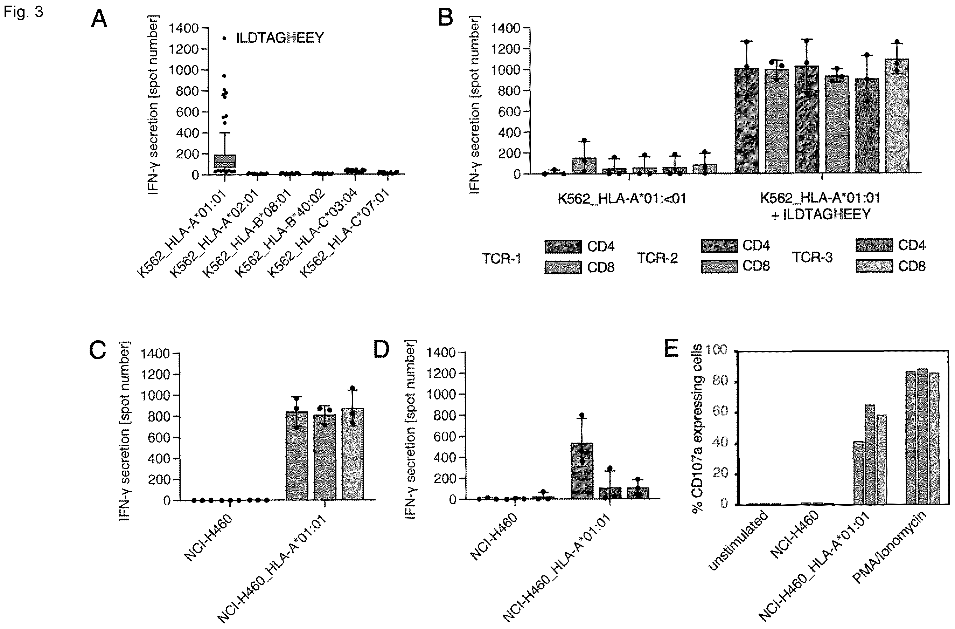

The term *having substantially the same biological activity* in the context of the present invention relates to either the main function of an isolated TCR having the CDR3 according to the invention, i.e. conferring specific, HLA-A*01:01 restricted reactivity to KRAS Q61H-peptide SEQ ID NO 021 to a T cell expressing this TCR. This can be confirmed, for example, by ELISpot analysis of a CD4, or CD8 T cell engineered to express a TCRα and TCRβ according to the invention, stimulated by HLA-transduced K562 cells pulsed with the peptide as indicated in Fig. 3 (B) of the Examples herein. T cell activation in a corresponding assay can be confirmed by at least 500 IFN-γ positive T cell clones.

### General Biochemistry: Peptides, Amino Acid Sequences

The term *polypeptide* in the context of the present specification relates to a molecule consisting of 50 or more amino acids that form a linear chain wherein the amino acids are connected by peptide bonds. The amino acid sequence of a polypeptide may represent the amino acid sequence of a whole (as found physiologically) protein or fragments thereof. The term "polypeptides" and "protein" are used interchangeably herein and include proteins and fragments thereof. Polypeptides are disclosed herein as amino acid residue sequences.

The term *peptide* or oligopeptide in the context of the present specification relates to a molecule consisting of up to 50 amino acids, in particular 8 to 30 amino acids, more particularly 8 to 15 amino acids, that form a linear chain wherein the amino acids are connected by peptide bonds.

Amino acid residue sequences are given from amino to carboxyl terminus. Capital letters for sequence positions refer to L-amino acids in the one-letter code (Stryer, Biochemistry, 3rd ed. p. 21). Lower case letters for amino acid sequence positions refer to the corresponding D- or (2R)-amino acids. Sequences are written left to right in the direction from the amino to the carboxy terminus. In accordance with standard nomenclature, amino acid residue sequences are denominated by either a three letter or a single letter code as indicated as follows: Alanine (Ala, A), Arginine (Arg, R), Asparagine (Asn, N), Aspartic Acid (Asp, D), Cysteine (Cys, C), Glutamine (Gln, Q), Glutamic Acid (Glu, E), Glycine (Gly, G), Histidine (His, H), Isoleucine (Ile, I), Leucine (Leu, L), Lysine (Lys, K), Methionine (Met, M), Phenylalanine (Phe, F), Proline (Pro, P), Serine (Ser, S), Threonine (Thr, T), Tryptophan (Trp, W), Tyrosine (Tyr, Y), and Valine (Val, V).

The term *Ras family protein* in the context of the present specification relates to the proteins encoded by one of three Ras genes. Four almost identical proteins are known: HRAS, NRAS, KRAS4A and KRAS4B. [i.e. Kirsten rat sarcoma viral oncogene homolog (KRAS), neuroblastoma RAS viral (v-ras) oncogene homolog (NRAS) and Harvey rat sarcoma viral oncogene homolog (HRAS)] encode four RAS proteins, with two KRAS isoforms that arise from alternative RNA splicing (KRAS4A and KRAS4B).

### General Molecular Biology: Nucleic Acid Sequences, Expression

The term *gene* refers to a polynucleotide containing at least one open reading frame (ORF) that is capable of encoding a particular polypeptide or protein after being transcribed and translated. A polynucleotide sequence can be used to identify larger fragments or full-length coding sequences of the gene with which they are associated. Methods of isolating larger fragment sequences are known to those of skill in the art.

The term *transgene* in the context of the present specification relates to a gene or genetic material that has been transferred from one organism to another. In the present context, the term may also refer to transfer of the natural or physiologically intact variant of a genetic sequence into tissue of a patient where it is missing. It may further refer to transfer of a natural encoded sequence the expression of which is driven by a promoter absent or silenced in the targeted tissue.

The term *recombinant* in the context of the present specification relates to a nucleic acid, which is the product of one or several steps of cloning, restriction and/or ligation and which is different from the naturally occurring nucleic acid. A recombinant virus particle comprises a recombinant nucleic acid.

The terms *gene expression* or *expression,* or alternatively the term *gene product,* may refer to either of, or both of, the processes - and products thereof - of generation of nucleic acids (RNA) or the generation of a peptide or polypeptide, also referred to transcription and translation, respectively, or any of the intermediate processes that regulate the processing of genetic information to yield polypeptide products. The term *gene expression* may also be applied to the transcription and processing of a RNA gene product, for example a regulatory RNA or a structural (e.g. ribosomal) RNA. If an expressed polynucleotide is derived from genomic DNA, expression may include splicing of the mRNA in a eukaryotic cell. Expression may be assayed both on the level of transcription and translation, in other words mRNA and/or protein product.

The term *Nucleotides* in the context of the present specification relates to nucleic acid or nucleic acid analogue building blocks, oligomers of which are capable of forming selective hybrids with RNA or DNA oligomers on the basis of base pairing. The term *nucleotides* in this context includes the classic ribonucleotide building blocks adenosine, guanosine, uridine (and ribosylthymine), cytidine, the classic deoxyribonucleotides deoxyadenosine, deoxyguanosine, thymidine, deoxyuridine and deoxycytidine. It further includes analogues of nucleic acids such as phosphothioates, 2'O-methylphosphothioates, *peptide nucleic acids* (PNA; N-(2-aminoethyl)-glycine units linked by peptide linkage, with the nucleobase attached to the alpha-carbon of the glycine) or *locked nucleic acids* (LNA; 2'O, 4'C methylene bridged RNA building blocks). Wherever reference is made herein to a *hybridizing sequence,* such hybridizing sequence may be composed of any of the above nucleotides, or mixtures thereof.

The term *nucleic acid expression vector* in the context of the present specification relates to a plasmid, a viral genome or an RNA, which is used to transfect (in case of a plasmid or an RNA) or transduce (in case of a viral genome) a target cell with a certain gene of interest, or -in the case of an RNA construct being transfected- to translate the corresponding protein of interest from a transfected mRNA. For vectors operating on the level of transcription and subsequent translation, the gene of interest is under control of a promoter sequence and the promoter sequence is operational inside the target cell, thus, the gene of interest is transcribed either constitutively or in response to a stimulus or dependent on the cell's status. In certain embodiments, the viral genome is packaged into a capsid to become a viral vector, which is able to transduce the target cell.

### (Cancer) Immunotherapy

In the context of the present specification, the term *adoptive cell therapy (ACT),* also used as *adoptive therapy* or *adoptive T-cell therapy* represents a recent immunotherapeutic approach within the realm of cancer treatment, which uses the potent anti-tumor capabilities of autologous (a patient's own) or allogeneic (another patient's) immune cells by engineering or activating the cells for enhanced tumor recognition and destruction. The process of autologous adoptive cell therapy begins with the extraction of specific immune cells, typically T cells, from a patient's bloodstream; alternatively, to develop an allogenic adoptive T cell the cells may be derived from a cell bank. These cells are then modified ex vivo through genetic engineering techniques or activated ex vivo through various stimulation methods, to enhance their tumor-specific recognition and cytotoxicity. The genetic modification involves the introduction of a T cell receptor (TCR) encoding transgene(s) can be transduced to equip the T cells with the ability to recognize tumor-specific Q61H antigens according to the invention. The engineered or activated T cells are then expanded to generate a substantial population, often referred to as a cell product. Once the cell product is prepared, it is infused into the patient. Adoptive therapy encompasses modalities such as Tumor-Infiltrating Lymphocyte (TIL) therapy; T-Cell Receptor (TCR) Therapy, which involves genetically modifying T cells to express TCRs specific for tumor antigens; Natural Killer (NK) Cell Therapy.

The term *cancer* as used in the context of the present specification relates to malignant neoplastic disease characterized by a RAS family protein having a Q61H mutation; the terms "cancer" and "malignant neoplastic disease" are used synonymously herein. They specifically include carcinoma (epithelial derived cancer), sarcoma (connective tissue derived cancer), lymphoma and leukemia, germ-cell derived tumors and blastomas. Particular alternatives of any of the aspects and embodiments disclosed herein are directed at the use of the compounds and compositions of the invention in treatment of solid tumors. Other alternatives of any of the aspects and embodiments disclosed herein are directed at the use of the combinations of the invention in treatment of liquid cancers such as myelogenous or granulocytic leukemia, particularly AML, lymphatic, lymphocytic, or lymphoblastic leukemia and lymphoma, polycythemia vera or erythremia.

As used herein, the term *treating* or *treatment* of any disease or disorder (e.g. cancer) refers in one embodiment to ameliorating the disease or disorder (e.g. slowing or arresting or reducing the development of the disease or at least one of the clinical symptoms thereof). In another embodiment "treating" or "treatment" refers to alleviating or ameliorating at least one physical parameter including those which may not be discernible by the patient. In yet another embodiment, "treating" or "treatment" refers to modulating the disease or disorder, either physically, (e.g., stabilization of a discernible symptom), physiologically, (e.g., stabilization of a physical parameter), or both. Methods for assessing treatment and/or prevention of disease are generally known in the art, unless specifically described hereinbelow.

As used herein, the term *pharmaceutical composition* refers to a compound of the invention, or a pharmaceutically acceptable salt thereof, together with at least one pharmaceutically acceptable carrier. In certain embodiments, the pharmaceutical composition according to the invention is provided in a form suitable for topical, parenteral or injectable administration.

As used herein, the term *pharmaceutically acceptable carrier* includes any solvents, dispersion media, coatings, surfactants, antioxidants, preservatives (for example, antibacterial agents, antifungal agents), isotonic agents, absorption delaying agents, salts, preservatives, drugs, drug stabilizers, binders, excipients, disintegration agents, lubricants, sweetening agents, flavoring agents, dyes, and the like and combinations thereof, as would be known to those skilled in the art (see, for example, Remington: the Science and Practice of Pharmacy, ISBN 0857110624). The invention also encompasses nanoparticles, liposomes, or cellular carriers within the meaning of *pharmaceutically acceptable carrier.*

### Detailed Description of the Invention

### T cell receptors

A first aspect of the invention relates to T cell receptors described by the inventors in the Examples herein comprising a TCRα polypeptide (also known as a TCR alpha chain), in association with a TCRβ polypeptide (also known as a TCR beta chain) conferring T cell specificity for peptides derived from a RAS family protein comprising a Q61H mutation presented by tumor cells in the context of an HLA-A*01:01 allele.

The TCRα polypeptide is characterized CDR3α sequence comprising the following sequence, or a variant CDR3α sequence comprising 1, or 2 amino acid substitutions compared to the following motif:
CALSEA(XXX)¹G(XX)²LX³F, wherein (XXX)¹ is NQA, GSS, or KAA, (XX)² is TA, SA, or NK, and X³ is I or T (SEQ ID NO 022).

The TCRβ polypeptide is characterized CDR3β sequence comprising the following sequence, or a variant CDR3β sequence comprising 1, or 2 amino acid substitutions compared to the following motif:
CASSR(XX)¹PP(XXXXX)²F, wherein (XX)¹ is KV, RW, RR or KL, and (XXXXX)² is VYGYT, VYGDT, TDTQY, or TYGYT (SEQ ID NO 023).

Such variant sequences compared to the most commonly isolated clones listed in Table 1 were observed in subsets of TCR of clones isolated from patients in the examples. In one example, a TCR was observed with a variant CDR3α having an I in position 3 of SEQ ID NO 022, instead of an L. Other clones were observed carrying a variant CDR3β with a non-conservative amino acid substitution, namely an M at position 3 of SEQ ID NO 023, instead of an S. TCR according to the invention are tolerant of small changes to the amino acid sequence in variant CDR3 according to this aspect of the invention.

In particular embodiments of the isolated TCR according to the invention, the CDR3α sequence, and/or the CDR3β sequence comprises a variant CDR3β sequence, and/or a variant CDR3α sequence having at most one amino acid substitution compared to SEQ ID NO 022, and/or SEQ ID NO 023, respectively.

In some embodiments of the isolated TCR according to the invention, the CDR3α sequence, and/or the CDR3β sequence comprises a variant CDR where said one, or two amino acid substitutions compared to SEQ ID NO 022, or SEQ ID NO 023 are selected according to the following substitution rules:
- glycine (G) and alanine (A) are interchangeable; valine (V), leucine (L), and isoleucine (I) are interchangeable, A and V are interchangeable;
- tryptophan (W) and phenylalanine (F) are interchangeable, tyrosine (Y) and F are interchangeable;
- serine (S) and threonine (T) are interchangeable;
- aspartic acid (D) and glutamic acid (E) are interchangeable
- asparagine (N) and glutamine (Q) are interchangeable; N and S are interchangeable; N and D are interchangeable; E and Q are interchangeable;
- methionine (M) and Q are interchangeable;
- cysteine (C), A and S are interchangeable;
- proline (P), G and A are interchangeable;
- arginine (R) and lysine (K) are interchangeable.

In particular embodiments of the isolated TCR according to the invention, the TCRα polypeptide comprises a variable domain selected from the list consisting of SEQ ID NO 011, SEQ ID NO 013, SEQ ID NO 015, SEQ ID NO 017, and SEQ ID NO 019. In other embodiments, the TCRα polypeptide comprises a variant variable domain sequence with ≥80%, ≥85%, ≥90%, ≥92%, ≥94%, ≥96%, ≥98%, or ≥99% sequence identity to SEQ ID NO 011, SEQ ID NO 013, SEQ ID NO 015, SEQ ID NO 017, and SEQ ID NO 019 (and having a CDR3α as specified above with at most one or two amino acid substitutions compared to SEQ ID NO 001, 003, 005, 007, or 009), and having substantially the same biological activity.

In certain embodiments of the isolated TCR according to the invention, the TCRβ polypeptide comprises SEQ ID NO 012, SEQ ID NO 014, SEQ ID NO 016, SEQ ID NO 018, and SEQ ID NO 020. In other embodiments, the TCRβ polypeptide comprises a variant variable domain sequence with a sequence with ≥80%, ≥85%, ≥90%, ≥92%, ≥94%, ≥96%, ≥98%, or ≥99% sequence identity to SEQ ID NO 012, SEQ ID NO 014, SEQ ID NO 016, SEQ ID NO 018, and SEQ ID NO 020, (and having a CDR3β as specified above with at most one or two amino acid substitutions compared to SEQ ID NO 002, 004, 006, 008, or 010) and having substantially the same biological activity.

Biological activity for the above variant TCR chains is defined as conferring T cell-specificity towards a Q61H mutant peptide presented by HLA-A*01:01 tumor cells, confirmed, for example, by means of an effector cytokine assay as provided in Fig. 3B of the examples.

In some embodiments of the isolated TCR according to the invention, the TCRα polypeptide and the TCRβ polypeptide comprise, or have variable domains consisting of: SEQ ID NO 011, and SEQ ID NO 012; SEQ ID NO 013, and SEQ ID NO 014; SEQ ID NO 015 and SEQ ID NO 016; SEQ ID NO 017 and SEQ ID NO 018; or SEQ ID NO 019 and SEQ ID NO 020, respectively.

In particular embodiments of the isolated TCR according to the invention, the TCRα polypeptide and the TCRβ polypeptide comprise a CDR3α sequence and CDR3β sequence comprising: SEQ ID NO 001, and SEQ ID NO 002; SEQ ID NO 003, and SEQ ID NO 004; SEQ ID NO 005 and SEQ ID NO 006; SEQ ID NO 007 and SEQ ID NO 008; or SEQ ID NO 009 and SEQ ID NO 010, respectively.

In particular embodiments of the TCR, the TCR chains comprise a CDR3α sequence and CDR3β sequence consisting of SEQ ID NO 001, and SEQ ID NO 002 respectively. In particular embodiments of the TCR, the TCR chains comprise a CDR3α sequence and CDR3β sequence consisting of SEQ ID NO 003, and SEQ ID NO 004 respectively. In particular embodiments of the TCR, the TCR chains comprise a CDR3α sequence and CDR3β sequence consisting of SEQ ID NO 005, and SEQ ID NO 006 respectively. In particular embodiments of the TCR, the TCR chains comprise a CDR3α sequence and CDR3β sequence consisting of SEQ ID NO 007, and SEQ ID NO 008 respectively. In particular embodiments of the TCR, the TCR chains comprise a CDR3α sequence and CDR3β sequence consisting of SEQ ID NO 009, and SEQ ID NO 010 respectively.

In other embodiments of the isolated TCR according to the invention, the TCRα polypeptide and the TCRβ polypeptide comprise a CDR3α sequence and CDR3β sequence consisting of a variant CDR3α sequence and/or a variant CDR3β sequence which have at most 2 amino acid substitutions per CDR3 compared to SEQ ID NO 001, and SEQ ID NO 002; SEQ ID NO 003, and SEQ ID NO 004; SEQ ID NO 005 and SEQ ID NO 006; SEQ ID NO 007 and SEQ ID NO 008; or SEQ ID NO 009 and SEQ ID NO 010.

In other embodiments of the isolated TCR according to the invention, the TCRα polypeptide and the TCRβ polypeptide comprise a CDR3α sequence and CDR3β sequence consisting of a variant CDR3α sequence and/or a variant CDR3β sequence which have at most one amino acid substitutions per CDR3 compared to SEQ ID NO 001, and SEQ ID NO 002; SEQ ID NO 003, and SEQ ID NO 004; SEQ ID NO 005 and SEQ ID NO 006; SEQ ID NO 007 and SEQ ID NO 008; or SEQ ID NO 009 and SEQ ID NO 010.

Such HLA restricted α/β-TCRs with specificity for shared tumor antigens are the starting point of important applications. TCR heterodimers are of use of transgenic receptors conferring specificity to tumor antigens to a recipient cell to provide a tumor mutation-specific T cell clone, or may be produced in a soluble form, and optionally multimerized, for example in a tetramer format.

In some embodiments, the TCR heterodimer is expressed by means of recombinant gene technology to provide an engineered cell, or a population of engineered cells expressing a TCR α chain in complex with a TCR β chain characterized by the CDR3 according to the first aspect of invention on the cell surface. In particular embodiments, the engineered cell is a T cell.

In additional embodiments of the claimed invention, the variable domain sequence of the full TCR sequences provided herein is used to convey specificity to a full TCR heterodimer or polypeptide as defined herein having a constant chain, for example, a mouse human chimera transgene, or completely human TCR complex expressed from a transgene.

Alternatively, soluble TCRs may be generated and multimerized (see reference 23). TCR multimers labeled with fluorochromes can be used to stain tumor cells isolated from tumor biopsies to identify Q61H target peptide.

Another aspect of the invention relates to nucleic acid sequences encoding the T cell receptor according to any one embodiments of the first aspect of the invention described in the preceding paragraphs.

Another aspect of the invention relates to a nucleic acid expression vector comprising nucleic acid sequences encoding the T cell receptor according to any one embodiments of the first aspect of the invention described in the preceding paragraphs.

In particular embodiments, the nucleic acid sequence, or the nucleic acid expression vector encodes a TCR comprising both TCRα polypeptide and a TCRβ polypeptide, respectively comprising a CDR3α sequence and CDR3β sequence consisting SEQ ID NO 001, and SEQ ID NO 002; SEQ ID NO 003, and SEQ ID NO 004; SEQ ID NO 005 and SEQ ID NO 006; SEQ ID NO 007 and SEQ ID NO 008; or SEQ ID NO 009 and SEQ ID NO 010, or a variant CDR3 pair having at most 1, or 2 amino acid substitutions (per CDR3) compared to the above pairs of CDR3.

In some embodiments of the TCR according to the invention, the alpha chain comprises a TRAV19*01 / -J15*01 variable domain and the beta chain variable domain is TRBV27*01 / - D1*01 / -J1-2*01 or TRBV27*01 / -D2*01 / -J2-3*01 or TRBV27*01 / -D2*01 / -J1-2*01. In other embodiments of the TCR according to the invention, the alpha chain comprises a TRAV19*01 / -J17*01 and the beta chain comprises a TRBV27*01 / -J1-2*01 variable domain.

### Engineered T cells, adoptive cell therapies

Another aspect of the invention relates to engineered T cells comprising the T cell receptor as specified in any one of the embodiments of the TCR (or the nucleic acids or vectors encoding the TCR) described in the section *T cell receptors* above. Another aspect of the invention relates to a preparation of engineered T cells comprising the T cell receptor as specified in any one of the embodiments of the TCR, i.e. an expanded population of T cell clones specific for a Ras family Q61H mutation.

Various means of creating adoptive cell therapy product from cancer-specific T cell receptors are known to the skilled person. An "off the shelf" TCRs in vector format can be used for transduction into autologous T cells obtained from cancer patients for immunotherapeutic intervention, or into a universal donor cell in an allogenic adoptive cell therapy approach. Various genetic engineering technologies (CRISPR/Cas9, TALEN, zinc finger nucleases) may be used to produce allogeneic cellular therapeutic products from healthy donors which are more readily available and at higher numbers than from most patients, providing a single product can be used for the treatment of several patients. Both autologous as well as allogeneic adoptive cell products expressing a TCR according to the current invention from a transgene or vector, can be genetically engineered to be less immunogenic (e.g. via knock-out of endogenous HLAs in the allogeneic setting), less prone to exhaustion/dysfunction (e.g. per knockout of checkpoint receptors), and less susceptible to induce Graft-versus-host disease (GvHD) or unpredictable cross-reactivity due to the knock-out of the endogenous TCRs.

In particular embodiments the adoptive cell therapy or engineered T cell according to this aspect of the invention, is derived from autologous or allogeneic CD4+ and CD8+ T cells with a α/β TCR comprising the indicated CDR3. In other embodiments, the engineered cell is a different adaptive or innate immune cell, like γδ-T cells, NKT cells, and NK cells expressing the TCR receptor according to the invention. The genetic engineering technologies mentioned above enable co-transduction of NK cells with TCRs and the CD3-signalling domains necessary for the activation of the cells upon TCR-engagement with MHC presenting a Q61H peptide.

In particular embodiments of the engineered T cell, or preparation of engineered cells, the T cell (or expanded T cell preparation) is an autologous T cell therapy, or an allogeneic T cell therapy.

In certain embodiments, the engineered T cell comprising a TCR having the specified CDR3 pair according to the invention is a cell obtained from another subject, particularly an HLA-matched subject (allogeneic adoptive T cell therapy). While depending on the quality of the HLA match, the risk of alloimmunity may be significant, the logistics and procedural advantages of having a large selection of pre-made TC preparations to select from may facilitate this therapy to a vastly larger patient community in comparison to the far higher costs and regulatory hurdles of a bespoke, patient-individual therapy.

In certain embodiments, the allogenic therapy is applied in combination with an immune suppressive drug, for example, a corticosteroid, cyclosporine or tacrolimus.

In certain embodiments, the T cell preparation according to the invention is obtained from the same patient (autologous adoptive T cell therapy). This method has the advantage of avoiding the risk of adverse reactions, particularly an allo-immune reaction driven by the endogenous T cell receptors of the engineered T cell preparation.

A further aspect of the invention relates to a pharmaceutical composition comprising the TCR as specified in any one of the embodiments (or the nucleic acids or vectors encoding the TCR) described in the section *T cell receptors* above.

Another aspect of the invention relates to a pharmaceutical composition comprising an engineered T cell comprising a TCR as specified in any one of the embodiments (or the nucleic acids or vectors encoding the TCR) described in the section *T cell receptors* above.

In particular embodiments of the engineered cell, or the pharmaceutical composition, they are provided for use in treating a patient diagnosed with cancer.

In particular embodiments of the engineered cell, or the pharmaceutical composition, they are provided for use in treating a cancer patient expressing the HLA-A*01:01 HLA allele.

In particular embodiments of the engineered cell, or the pharmaceutical composition, they are provided for use in treating a patient diagnosed with a form of lung cancer. In particular embodiments, the cancer is NSCLC. IN other embodiments, the cancer is a metastases derived from a lung cancer.

In particular embodiments of the engineered cell, or the pharmaceutical composition, they are provided for use in treating a patient diagnosed with a form of gastrointestinal cancer. In particular embodiments, the cancer is a colorectal cancer.

In other embodiments of the engineered cell, or the pharmaceutical composition, they are provided for use in treating a patient diagnosed with a form of adenocarcinoma.

In other embodiments of the engineered cell, or the pharmaceutical composition, they are provided for use in treating a patient diagnosed with melanoma. In certain embodiments, the cancer patient has a metastasis of a melanoma.

In particular embodiments of the engineered cell, or the pharmaceutical composition, they are provided for use in treating a cancer patient wherein tumor tissue obtained from the patient is characterized by a Ras family protein with a Q61H mutation. HRAS, KRAS, or NRAS have all been found to carry Q61H mutations targeted by TCR according to the invention in a patient tissues derived from various cancers including, but not limited to, gastrointestinal and lung adenocarcinomas, and melanoma.

In certain embodiments of the engineered cell, or the pharmaceutical composition, they are provided for use in treating a cancer patient wherein tumor tissue obtained from the patient is characterized by a HRAS protein with a Q61H mutation.

In certain embodiments of the engineered cell, or the pharmaceutical composition, they are provided for use in treating a cancer patient wherein tumor tissue obtained from the patient is characterized by a KRAS protein with a Q61H mutation.

In certain embodiments of the engineered cell, or the pharmaceutical composition, they are provided for use in treating a cancer patient wherein tumor tissue obtained from the patient is characterized by a NRAS protein with a Q61H mutation.

### Cancer patient stratification and monitoring

A further aspect of the invention relates to a method of determining the likelihood of a cancer patient of having a favorable response to treatment with a soluble form of TCR, an engineered T cell, or pharmaceutical composition according to any one of the aspects or embodiments of the invention listed above. The method comprises the following steps:
Firstly, determining in a tumor nucleic acid sample obtained from said cancer patient (for example, nucleic acids isolated from liquid tumor biopsy or a formalin-fixed biopsy tissue sample) whether the patient's tumor is characterized by a HRAS, KRAS, or NRAS protein having a Q61H mutation. The method by which this characterization is achieved is not particularly limited, and may be achieved, for example, by performing a PCR with primer specific for the Q61H mutation, using a microarray with probes specific for a variety of common tumor mutations, or by sequencing reaction performed on genomic DNA, or cDNA obtained from mRNA).

Secondly, assigning the cancer patient a high likelihood of favorable response to treatment if the nucleic acid sample is characterized by a HRAS, KRAS, or NRAS protein having a Q61H mutation, which is targeted by TCR according to the invention.

In particular embodiments, the method further comprises a step of performing an HLA-typing, wherein the patient is assigned a high likelihood of favorable response to treatment if the patient is characterized by an HLA-A*01:01 HLA allele in addition to the presence of a Q61H mutation.

In particular embodiments, the method for stratifying cancer patients comprises a step of determining in a nucleic acid sample obtained said cancer patient (for example, nucleic acids isolated from liquid tumor biopsy, a formalin-fixed biopsy tissue sample, a blood sample, or any DNA sample) whether a nucleic acid encoding the isolated T cell receptor according to any one of the claims 1 to 4 is present in the nucleic acid sample. Here, the method is not particularly limited, and may be achieved, for example, by microarray, a sequencing reaction, particularly a next generation single cell sequencing reaction as demonstrated in the examples, or performing a PCR with a nucleic acid probe specific for the CDR3, or variable domains according to the invention on genomic DNA, or a cDNA library amplified with T cell receptor-specific primers. Subsequently in the assignment step, assigning the cancer patient a high likelihood of favorable response to treatment if a nucleic acid encoding at least one CDR3 selected from the list consisting SEQ ID NO 001-010 (or a variant having 1, or 2 amino acid substitutions) is present in the sample, indicating a response to the mutation is already ongoing in the patient.

These steps and optional embodiments can be combined in different ways apparent to the skilled person, for example a clinician to identify patients who will benefit from adoptive cell therapies comprising these TCR. To give one example of the method according to the invention incorporating some of the embodiments described above, firstly a patient blood sample is subjected to HLA-typing, showing the patient expresses the HLA A* 01:01 allele. A patient tumour sample is obtained, and subjected to mRNA sequencing, showing high expression by the tumour of a KRAS family protein comprising Q61H, indicating the patient expresses the antigen target of an engineered T cell according to the invention. In some embodiments, the mRNA tumour sequencing is also examined for the presence of nucleic acids encoding at least one CDR3 selected from the list consisting SEQ ID NO 001-010 (or a variant having 1, or 2 amino acid substitutions), indicating the patient has already initiated a T cell response against the Q61H mutation, which may be supplemented by additional engineered T cell clones to enhance the response.

A further aspect of the invention relates to a method of monitoring the treatment efficacy of a cancer patient having been administered an adoptive T cell therapy comprising an adoptive T cell therapy having a T cell receptor according to any one of the claims 1 to 4. The method comprises firstly, determining an expression level of a nucleic acid encoding at least one CDR3 selected from the list consisting SEQ ID NO 001-010 (or a variant having 1, or 2 amino acid substitutions) present in a nucleic acid sample obtained from said cancer patient after commencement of treatment, and

In a second step, comparing said expression level to an expression level of the CDR3 in a negative control to determine if the response has become greater over time. In some embodiments, the control is a baseline nucleic acid sample obtained from said cancer patient before commencement of treatment. In other embodiments, the control is a negative sample of the sample type of tissue, obtained from a patient who does not have a CDR3 according to the invention in their T cell repertoire. Alternatively, the negative control can be replaced by a negative threshold value reflecting the results obtained from such controls.

In some embodiments of the patient stratification method, or the monitoring method the nucleic acid sample in which the TCR will be measured has been obtained from a tumor sample, or blood sample. In particular embodiments of the patient stratification method, or the monitoring method the nucleic acid sample in which the TCR will be measured has been obtained from a blood sample.

Another aspect of the invention is a kit comprising nucleic acid probes specific to a sequence selected from the list consisting of SEQ ID NO 001 to 010 for use in a method to stratify patients, or a method to monitor the anti-tumor T cell response of a patient having received an adoptive cell transfer therapy according to the invention.

In particular embodiments, the kit comprises a primer set for amplifying and subsequent sequencing of SEQ ID NO 001-010. The kit is compatible with a sample for sequencing obtained from a tumor sample or a blood sample, as the inventors found the specific T-cells of interest to be present in the blood or tumor.

### Pharmaceutical Compositions, Administration/Dosage Forms and Salts

According to one aspect of the engineered cell according to the invention, it is provided as a pharmaceutical composition, pharmaceutical administration form, or pharmaceutical dosage form, said pharmaceutical composition, pharmaceutical administration form, or pharmaceutical dosage form comprising at least one pharmaceutically acceptable carrier, diluent or excipient.

In certain embodiments of the invention, the compound of the present invention is typically formulated into pharmaceutical dosage forms to provide an easily controllable dosage of the drug and to give the patient an elegant and easily handleable product.

The invention further encompasses a pharmaceutical composition comprising an engineered cell of the present invention, and a pharmaceutically acceptable carrier. In further embodiments, the composition comprises at least two pharmaceutically acceptable carriers, such as those described herein.

Certain embodiments of the invention relate to a dosage form for parenteral administration, such as subcutaneous, intravenous, intrahepatic or intramuscular injection forms. Optionally, a pharmaceutically acceptable carrier and/or excipient may be present.

The dosage regimen for the compounds of the present invention will vary depending upon known factors, such as the pharmacodynamic characteristics of the particular agent and its mode and route of administration; the species, age, sex, health, medical condition, and weight of the recipient; the nature and extent of the symptoms; the kind of concurrent treatment; the frequency of treatment; the route of administration, the renal and hepatic function of the patient, and the effect desired.

The pharmaceutical compositions of the present invention can be subjected to conventional pharmaceutical operations such as sterilization and/or can contain conventional inert diluents, lubricating agents, or buffering agents, as well as adjuvants, such as preservatives, stabilizers, wetting agents, emulsifiers and buffers, etc. They may be produced by standard processes, for instance by conventional mixing, granulating, dissolving or lyophilizing processes. Many such procedures and methods for preparing pharmaceutical compositions are known in the art, see for example L. Lachman et al. The Theory and Practice of Industrial Pharmacy, 4th Ed, 2013 (ISBN 8123922892).

### Method of Manufacture and Method of Treatment according to the invention

The invention further encompasses, as an additional aspect, the use of an engineered T cell as identified herein as specified in detail above, for use in a method of manufacture of a medicament for the treatment or prevention of a form of cancer having a Ras family protein with a Q61H mutation.

Similarly, the invention encompasses methods of treatment of cancer, comprising administering to a patient in need thereof a therapeutically effective amount of an engineered cell, as specified in detail herein.

The invention encompasses a method of treating a cancer patient with a tumor characterized by a Ras family protein with a Q61H mutation, the method comprising administering an effective dose of an adoptive T cell therapy characterized by an isolated T cell receptor according to any of the embodiments or aspects of the invention listed above.

The invention further encompasses the use of SEQ ID NO 001-010 identified herein for use in the manufacture of a kit for the detection of T cell responses to a Q61H mutation in a cancer patient's tumor.

The invention further encompasses a Biomarker composition comprising nucleic acid probes specific to SEQ ID NO 001-010. The invention further encompasses a method of providing information for diagnosis, or monitoring a patient T cell response, or adoptive T cell therapy outcome comprising nucleic acid probes specific to SEQ ID NO 001-010.

Wherever alternatives for single separable features are laid out herein as "embodiments", it is to be understood that such alternatives may be combined freely to form discrete embodiments of the invention disclosed herein. Thus, any of the alternative embodiments for a CDR3α may be combined with any of the alternative embodiments of CDR3α and these combinations may be combined with any engineered cell, or use for treating a subset of cancer patients mentioned herein.

The invention is further illustrated by the following examples and figures, from which further embodiments and advantages can be drawn. These examples are meant to illustrate the invention but not to limit its scope.

### Description of the Figures

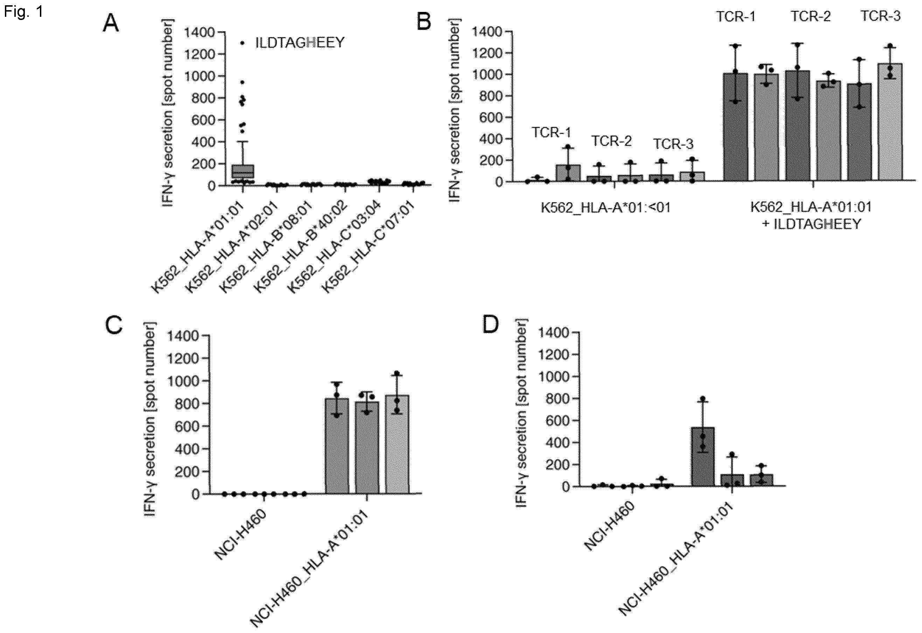

- Fig. 1: shows TCR-T cells transduced with TCRs. TCR-1, -2, -3 recognize the naturally processed and presented KRAS Q61H-peptide 55-64 (ILDTAGHEEY SEQ ID NO 021). (A) Identification of the KRAS Q61H-peptide as target antigen of TCR-V2-T cells. ELISpot analysis testing TCR-T cells against monoallelic P18-HLA transduced K562 cells pulsed with 96 candidate neoantigen peptides identified by WES- and RNA-Seq. Only K562/HLA-A*01:01 cells were recognized when pulsed with several synthetic peptides in a cross-reactive manner, though less strong as the KRAS Q61H-peptide. (B) K562/HLA-A*01:01 cells were recognized by TCR-1, -2, and -3-transduced CD4- and CD8-positive TCR-T cells when pulsed with mutant KRAS-peptide 55-64. (C, D) Recognition of KRAS Q61H-mutated, HLA-A*01:01-transduced cell line NCI-H460/HLA-A*01:01 in comparison to wildtype NCI-H460 by CD8-positive (C) and CD4-positive (D) TCR-T cells.

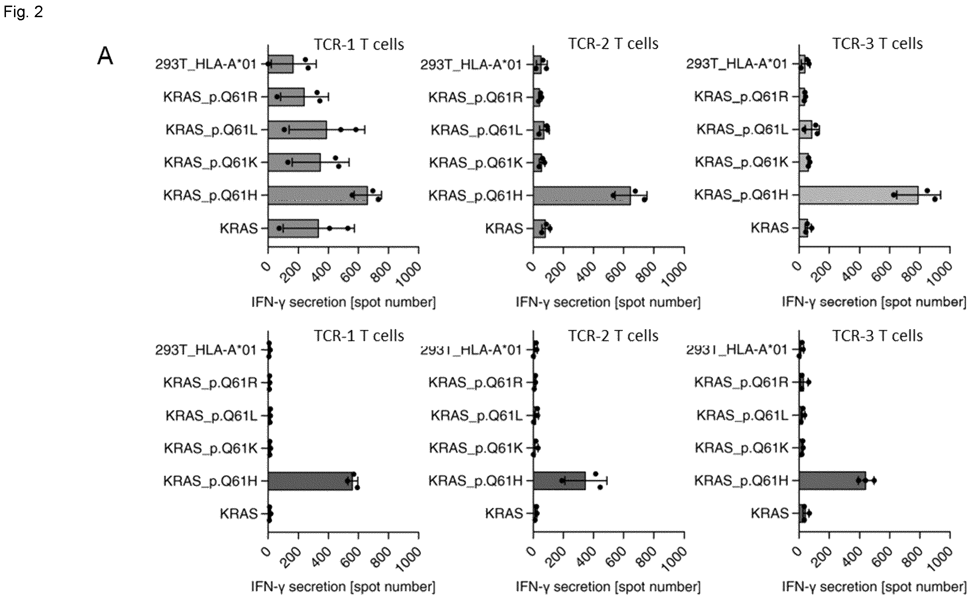

- Fig. 2: shows TCR-T cells transduced with TCRs -1, -2, -3 are KRAS Q61H-specific. (A) Reactivity of TCR-1/2/3 transduced CD8-positive (top row) and CD4-positive (bottom row) TCR-T cells against 293T cells transiently transfected with depicted (naturally occurring) KRAS-encoding variants and HLA-A*01:01. TCR-transduced CD8- and CD4-positive TCR-T cells showed exclusive recognition of KRAS Q61H-/HLA-A*01:01-expressing 293T cells.
- Fig. 3: TCR-T cells transduced with TCRs TCR-1, -2, -3 recognize the naturally processed and presented KRAS Q61H-peptide 55-64 (ILDTAGHEEY). (A) Identification of the KRAS Q61H-peptide as target antigen of TCR-2-T cells. ELISpot analysis testing TCR-T cells against monoallelic P18-HLA transduced K562 cells pulsed with 96 candidate neoantigen peptides identified by WES- and RNA-Seq. Only K562/HLA-A*01:01 cells were recognized when pulsed with several synthetic peptides in a cross-reactive manner, though less strong as the KRAS Q61H-peptide. (B) K562/HLA-A*01:01 cells were recognized by TCR-1, -2, and -3-transduced CD4- and CD8-positive TCR-T cells when pulsed with mutant KRAS-peptide 55-64 (color code of the legend used for all figures). (C,D) Recognition of KRAS Q61H-mutated, HLA-A*01:01-transduced cell line NCI-H460/HLA-A*01:01 in comparison to wildtype NCI-H460 by CD8-positive (C) and CD4-positive (D) TCR-T cells. (E) Flow cytometry showing degranulation (CD107a) as surrogate for lytic activity of TCR-1, -2, and -3-expressing CD8-positive TCR-T cells upon co-culture with NCI-H460/HLA-A*01:01.

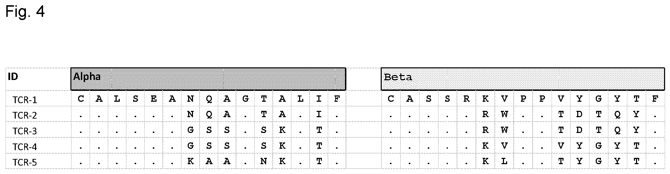

- Fig. 4: shoes TCRs 1-5 shown as multiple sequence alignments. Identical amino acids are depicted as dots.

### Examples

### Example 1: identification of T cell clonotypes with reactivity against lung adenocarcinoma

Tumor infiltrating lymphocyte TCRs were identified via an antigen-agnostic approach for identifying tumor-specific T-cell clonotypes by comparing high-throughput TCR-repertoire profiles of tumor and adjacent normal tissue-infiltrating T-cell clonotypes from surgical specimens obtained from lung adenocarcinoma patients according to previously described methods. When a T-cell infiltrates the tumor and provokes a specific receptor mediated interaction with a tumor antigen, this encounter is followed by activation, proliferation and enrichment of the clone in the tumor. Thus, the preferred localization of this unique TCR clonotype, as determined quantitatively by the ratio of the TCR clonotype frequencies between tumor and adjacent non-tumor tissue is a predictor of tumor-specificity. This technology is described in WO 2017/025564 A1. If such a unique tumor-specific TCR clonotype or structurally closely related TCR clonotypes, referred to as a TCR cluster, are detected in the tumors of other patients, this is indicative of the existence of a shared tumor antigen in these patients. This is particularly informative when TCR clusters are detected in HLA-matched patients revealing the nature of the HLA allele presenting the shared antigenic epitope. Identical or nearly identical tumor-specific TCRs identified among several patients of the same HLA-type showed reactivity against lung adenocarcinoma.

**Table 1. TCR isolated from one or more NSCLC patient samples from FFPE archives.**

| **ID** | **Alpha chain** | **Beta chain** |
|---|---|---|
| TCR-1 | TRAV19*01 /-J15*01 | TRBV27*01 / -D1*01 / -J1-2*01 |
| | CALSEANQAGTALIF SEQ ID NO 001 | CASSRKVPPVYGYTF SEQ ID NO 002 |
| TCR-2 | TRAV19*01 / -J15*01 | TRBV27*01 / -D2*01 / -J2-3*01 |
| | CALSEANQAGTALIF SEQ ID NO 003 | CASSRRWPPTDTQYF SEQ ID NO 004 |
| TCR-3 | TRAV19*01 /-J17*01 | TRBV27*01 /-J1-2*01 |
| | CALSEAKAAGNKLTF SEQ ID NO 005 | CASSRKLPPTYGYTF SEQ ID NO 006 |
| TCR-4 | TRAV19*01 /-J15*01 | TRBV27*01 / -D2*01 / -J1-2*01 |
| | CALSEAGSSGSKLTF SEQ ID NO 007 | CASSRRWPPTDTQYF SEQ ID NO 008 |
| TCR-5 | TRAV19*01 /-J15*01 | TRBV27*01 / -D2*01 / -J1-2*01 |
| | CALSEAGSSGSKLTF SEQ ID NO 009 | CASSRKVPPVYGYTF SEQ ID NO 010 |

### Production and functional characterization of tumor-specific TCR-T cells

Tumor-specific TCR-T cells were produced by retroviral transduction of donor-derived T cells with synthesized codon-optimized sequences encoding TCR-V1-V4 (Table 2.) as described (18). Before retroviral transduction, the recipient T cells were depleted from endogenous TCRs by CRISPR/CAS9-mediated knock-out (KO) of human (h)TRBC and TRAC domains to prevent mispairing of endogenous and recombinant TCR chains with unpredictable adverse specificities or allo-reactivity against allogeneic antigen-presenting cells used for subsequent antigen-screening. Successful hTCR-KO and expression of recombinant cTCRs was confirmed by flow cytometry, and IFN-γ-ELISpot-assays with the cTCR-T cells showed recognition of HLA-matched tumor cells. OKT-3 mAb was used for unspecific activation control. Pan-HLA class I mAb W6/32 blocked tumor-recognition in all experiments.

**Table 2. TCR sequences of tumor-specific TCR-T cells.**

| **ID** | **Alpha chain** | **Beta chain** |
|---|---|---|
| TCR-1 | TRAV19*01 /-J15*01 | TRBV27*01 / -D1*01 / -J1-2*01 |
| | SEQ ID NO 011 KVTQAQTEISVVEKEDVTLDCVYETRDTTYYLF WYKQPPSGELVFLIRRNSFDEQNEISGRYSWNF QKSTSSFNFTITASQVVDSAVYFCALSEANQAG TALIFGKGTTLSVSS | SEQ ID NO 012 EAQVTQNPRYLITVTGKKLTVTCSQNMNHEY MSWYRQDPGLGLRQIYYSMNVEVTDKGDVP EGYKVSRKEKRNFPLILESPSPNQTSLYFCASSR KVPPVYGYTFGSGTRLTVV |
| TCR-2 | TRAV19*01 / -J15*01 | TRBV27*01 / -D2*01 / -J2-3*01 |
| | SEQ ID NO 013 | SEQ ID NO 014 |
| | KVTQAQTEISVVEKEDVTLDCVYETRDTTYYLF WYKQPPSGELVFLIRRNSFDEQNEISGRYSWNF QKSTSSFNFTITASQVVDSAVYFCALSEANQAG TALIFGKGTTLSVSS | EAQVTQNPRYLITVTGKKLTVTCSQNMNHEY MSWYRQDPGLGLRQIYYSMNVEVTDKGDVP EGYKVSRKEKRNFPLILESPSPNQTSLYFCASSR RWPPTDTQYFGPGTRLTVL |
| TCR-3 | TRAV19*01 / -J17*01 | TRBV27*01 / -J1-2*01 |
| | SEQ ID NO 015 AQKVTQAQTEISVVEKEDVTLDCVYETRDTTYY LFWYKQPPSGELVFLIRRNSFDEQNEISGRYSW NFQKSTSSFNFTITASQVVDSAVYFCALSEAKA AGNKLTFGGGTRVLVKP | SEQ ID NO 016 EAQVTQNPRYLITVTGKKLTVTCSQNMNHEY MSWYRQDPGLGLRQIYYSMNVEVTDKGDVP EGYKVSRKEKRNFPLILESPSPNQTSLYFCASSR KLPPTYGYTFGSGTRLTVV |
| TCR-4 | TRAV19*01 / -J15*01 | TRBV27*01 / -D2*01 / -J1-2*01 |
| | SEQ ID NO 017 AQKVTQAQTEISVVEKEDVTLDCVYETRDTTYY LFWYKQPPSGELVFLIRRNSFDEQNEISGRYSW NFQKSTSSFNFTITASQVVDSAVYFCALSEAGSS GSKLTFGGGTRVLVKP | SEQ ID NO 018 EAQVTQNPRYLITVTGKKLTVTCSQNMNHEY MSWYRQDPGLGLRQIYYSMNVEVTDKGDVP EGYKVSRKEKRNFPLILESPSPNQTSLYFCASSR RWPPTDTQYFGPGTRLTVL |
| TCR-5 | TRAV19*01 /-J15*01 | TRBV27*01 / -D2*01 / -J1-2*01 |
| | SEQ ID NO 019 AQKVTQAQTEISVVEKEDVTLDCVYETRDTTYY LFWYKQPPSGELVFLIRRNSFDEQNEISGRYSW NFQKSTSSFNFTITASQVVDSAVYFCALSEAGSS GSKLTFGGGTRVLVKP | SEQ ID NO 020 EAQVTQNPRYLITVTGKKLTVTCSQNMNHEY MSWYRQDPGLGLRQIYYSMNVEVTDKGDVP EGYKVSRKEKRNFPLILESPSPNQTSLYFCASSR KVPPVYGYTFGSGTRLTVV |

### Example 2: Target antigen screening using tumor-specific TCR-T cells

Neoantigens have been associated with favorable clinical responses to immunotherapy in NSCLC, indicating the existence of specific immunity in responding patients. Comparative whole exome- and transcriptome sequencing of tumor and adjacent lung tissue samples were carried out to identify tumor-specific non-synonymous variants as neoantigen. 73 expressed non-synonymous single nucleotide variants (SNV) and one frameshift-mutation were identified. Binding predictions of mutated candidate peptides to patient HLA I alleles (HLA-A*01:01/*02:01, HLA-B*08:01/*40:02, HLA-C*03:04/*07:01) using IEDB (http://tools.iedb.org/mhci/) and NetMHC4.0 public databases found 581 9- or 10-mer peptides with IC50 <500nM and/or percentile rank <6. HLA allele-assorted peptides were affinity score-ranked, and the 96 top-scoring peptides were synthesized and tested for recognition. K562 cells transduced with HLA I alleles were pulsed with candidate peptides and tested for recognition by TCR-T cells using ELISpot assays. TCR-T cells expressing TCR-V1, -V2, and -V3 all responded to KRAS Q61H peptide 55-64 ILDTAGHEEY (SEQ ID NO 21), irrespective whether CD4- or CD8-positive lymphocytes expressed the TCRs (Fig. 1A, B).

### Recognition of the naturally processed and presented KRAS Q61H-peptide

To verify that the mKRAS peptide is processed and presented, NCI-H460 cells were tested for recognition. NCI-H460 cells are natural carriers of the KRAS Q61H-encoding mutation (KRAS c.183A>T) but are negative for HLA-A*01:01 and were thus transduced with this allele. Wildtype NCI-H460 and NCI-H460/HLA-A*01:01 cells were tested for TCR-T-cell recognition by ELISpot (Fig. 1D,E). While NCI-H460 cells induced no response as expected, NCI-H460/HLA-A*01:01 cells were strongly recognized by CD8-positive TCR-T cells transduced with any of the three TCRs (Fig. 3C, E). In contrast, CD4-positive TCR-T cells showed significant reactivity only when transduced with TCR-V1 (Fig. 3D). H-, K-, and NRAS protein-family members share identical Aa sequences from position 1 to 84, implying that the target peptide (Aa 55-64 SEQ ID NO 021) can be processed and presented from any of these proteins. Variations at mutation hotspot Aa-position 61 in RAS comprise Q61H, Q61R, Q61K, and Q61L occurring with varying frequencies in different tumor entities. Taking advantage of three independently evolved KRAS Q61H-specific TCRs with significant Aa variations in the peptide-binding CDR3 domains (Fig. 1F), the inventors tested whether any of the TCRs was capable of cross-reacting against the alternative mutations. ELISpot assays with 293T cells transfected with HLA-A*01:01 and cDNAs encoding all four possible mKRAS variants as well as wildtype KRAS, showed that all three TCRs are specific for the Q61H mutation (Fig. 2A). The results demonstrate the perfect specificity of the TCR family of Table 1 for the KRAS Q61 H neoepitope.

### Discovery of matching TCR-sequences in KRAS Q61H-positive tumors of HLA-A*01:01-positive patients

Consistent with KRAS Q61H being a cancer driver in NSCLC, the hotspot variant was clonal and detected in genomic DNA from a tumor relapse obtained 32 months after surgery of the primary tumor. TCR-repertoire profiling using template DNA from the FFPE sample detected multiple clonotypes predicted tumor-specific from the primary tumor, including confirmed tumor-reactive TCRs at high frequencies (Fig. 3A).

To further investigate the immunogenicity of the KRAS Q61H mutation, the inventors performed TCR-profiling on FFPE-samples from 29 patients with various KRAS Q61H-positive tumors including 14 lung cancers, nine gastro-intestinal (including CRCs, pancreas, and bile duct cancers), and six un-specified tumors. The KRAS-mutation was encoded by SNVs c.183A>T in 19 and c.183A>C in ten cases. Repertoire-analysis of TRBV- and TRAV-sequences revealed CDR3 sequences highly related (for example, leucine/isoleucine interchangeably present in the same CDR3 position) or even identical clones listed in Table. 1 in six out of the 29 patient samples analyzed (in 4/14 lung cancers). Taken together, these results suggest a convergent selection of cognate immune receptors in different patients with KRAS Q61H-positive cancers, indicating a high epitope immunogenicity.

### Summary

Engineered TCR-T cells recognized autologous tumor cells and HLA- and KRAS-mutation-matched tumor cell lines. Screening for recognition of expressed non-synonymous neoepitopes and overexpressed TAA candidates revealed that the selected TCRs specifically recognized mutant (m)KRAS Q61H-peptide 55-64 (ILDTAGHEEY, highlighted amino acid substitution, SEQ ID NO 021) presented by HLA-A*01:01.The potential therapeutic benefit of the selected TCRs is supported by a) functional characterization of TCR-T cells genetically engineered with the TCRs, b) phenotypic characterization of the original TIL clonotypes, c) the presence of these clonotypes in tumor relapse acquired more than 30 months after primary tumor surgery, and d) the discovery of identical or highly homologous TCRs in six out of 29 archival (FFPE) tumor samples with confirmed Q61H mutations. The mKRAS-reactive TCRs described here can be used as off-the-shelf TCRs for TCR-T therapy in HLA-A*01:01-positive patients with m(K/H/N)RAS Q61H-positive tumors.

### Material and methods

### TCR repertoire profiling of tumor- and lung-infiltrating lymphocytes and TIL-scRNA-Seq

The results of TCR repertoire sequencing (TRBV chains, 2x 150bp paired Illumina reads) were processed by in-house developed software using both reads to build a consensus sequence covering the complete CDR3 region and removing inconsistent non-overlapping read-pairs. High-quality consensus CDR3 sequences were clustered into unique clonotypes, respective V- and J-segment IDs, and a clonotype frequency was calculated as percentage of clonotype reads compared to all sample reads. Clonotype sequences were further analyzed for productive ORFs discarding non-functional sequences. The output frequency matrix with each row belonging to a unique CDR3 nucleotide sequence (Table 1) showed clonotype frequencies in peripheral blood, tumor and adjacent non-tumor tissues. A frequency ratio TIL/adjacent normal lung was calculated for all clonotypes; those with a ratio >5 were enriched for tumor-specific T-cells (patents EP3180433A1, EP3372683A1). From sorted subpopulations of TILs, lung-infiltrating lymphocytes, PBMCs, and blood plasma, (g)DNA was isolated and subjected to proprietary TCRSafe^{®}-analysis using human TRBV/J-specific primer sets (16). Briefly, gDNA from CD3+, CD8+, and PD1+ T-cell subpopulations was isolated using the QIAamp blood kit (Qiagen) and NGS libraries were generated employing a two-step PCR protocol. gDNA from FFPE samples processed with the AllPrep DNA/RNA FFPE kit (Qiagen) and from urine and plasma with the Norgen Plasma/Serum RNA/DNA Purification Mini Kit (BioCat GmbH) was applied to TCRSafe^{®}-analysis, too. In addition to TRBV-sequencing, FFPE-samples were subjected also to TCRSafe^{®}-analysis using human TRAV/J-specific primer sets. Single cell cDNA-libraries were generated from CD3+ TIL single cell suspensions using 10x Genomics^{®} GemCodeTM Technology (10x Genomics B.V.). Briefly, lymphocytes were processed using the 10x Genomics Chromium Next GEM Single Cell V(D)J Reagent Kit in combination with the Chromium Single Cell V(D)J Enrichment Kit (Human) according to protocol. After clean-up, libraries were analyzed by Illumina next generation sequencing (StarSEQ GmbH).

### CRISPR/CAS9 engineering of primary T cells and cell lines

T cells isolated from Buffy Coats from three healthy donors were isolated by Ficoll (Sigma-Aldrich) separation and MACS-sorting according to protocol (Miltenyi Biotec). After OKT3-activation (plate-bound, 30 ng/µl), T cells were subjected to CRISPR/CAS9-mediated knockout of endogenous TCRs. Ribonucleoprotein (RNP) complexes were delivered by Human T Cell Nucleofector^{™} Kit (Lonza). Both TCR chains were targeted by two crRNAs. The TRBC-crRNA was previously described (17), the TRAC-crRNA was designed using the Alt-R Custom Cas9 crRNA Design Tool (IDT). Combined at 1:1-ratio with the Alt-R^{®} CRISPR-Cas9 tracrRNA (IDT) two different gRNA complexes were formed. gRNA complexes were combined with recombinant Cas9 (IDT) for RNP complex generation (20 min, RT). 4x10⁶ T cells were transfected in Nucleofector^{®} Solution supplemented with 1 µM Alt-R^{®} Cas9 electroporation enhancer (IDT) and 4 µM of RNPs. T cells were electroporated with program T-023 on a Nucleofector^{™} 2b Device (Lonza). T cells were cultivated at 1x10⁶ cells/ml Panserin complete (plus 600 U/ml IL-2). TCR-KO efficiency was assessed 4-6 days later via flow cytometry.

### TCR-encoding DNA-synthesis and cloning

T-cell receptor alpha-chain (TRAV/J-) and TCR beta-chain (TRBV/D/J-) region-coding sequences were synthesized as G-blocks (IDT) and cloned as bicistronic constructs connected by a P2A-encoding linker into gamma-retroviral expression vector pMX-puro as described ⁵⁴. TCRs were designed as chimeric constructs in which the human TRA- and TRB-constant-region sequences (TRAC, TRBC) were replaced by murine homologous sequences.

### Stable transduction of primary T cells and cell lines

TCR-encoding γ-retroviral particles were produced for transduction of primary T cells as described (18, 19) T cells from Buffy coats of healthy donors were isolated by Ficoll separation followed by CD8- and CD4-magnetic bead isolation according to protocol (Miltenyi Biotec). After activation with plate-bound OKT-3 (30ng/µl) and culture for 3-5 days, endogenous TRAC/TRBC-knockout was accomplished. Viral particles were produced using Phoenix-ampho packaging cells seeded at 1,3x10e6 cells per 100mm plate. After 24 h, cells were co-transfected with 5 µg each pCOLT-GALV, pHIT60 and 10 µg pMX/TCR using Fugene-6 according to protocol (Promega). Transfection medium was changed for T-cell medium after 24h and supernatant was harvested at 16 h following cell-pelleting. TCR-T cells were generated by spin-inoculation with retrovirus-containing T cell medium of 2x10e6 T cells per reaction and TCR-T cells expanded and selected using puromycin (1µg/ml, Sigma Aldrich). Transductions of K562- and NCI-H460 cells were performed with pMX/HLA-constructs.

### Culture of cell lines and primary T cells

Cell lines and primary T cells were grown in incubators at 37°C, 5% CO₂, >85% humidity. HEK 293T-, K562-, NCI-H460-, and MZ-LC-16 were maintained in RPMI-1640 supplemented with 10% FBS, and 1% penicillin/streptomycin.HLA-monoallelic K562 cells were engineered to express all six HLA-alleles of P18, and NCI-H460 to express HLA-A*01:01 using γ-retroviral transduction (see below). Cells were maintained in RPMI+ plus puromycin (1µg/ml, Sigma Aldrich). Primary T cells from healthy donors were grown in Panserin-413 (PAN-Biotech, Aidenbach, Germany) supplemented with 10% heat-inactivated pooled human serum (provided by the blood bank of UMC Mainz, Germany), 1% Penicillin/Streptomycin (Sigma Aldrich), and rhIL-2 (250-600 IU/mL Novartis, Basel, Switzerland). Cell lines were regularly subjected to mycoplasma testing to ensure absence of contamination.

### Transient transfection of HEK 293T cells

Overexpressed antigen and KRAS-encoding cDNAs were reverse-transcribed and amplified from P18-tumor- and NCI-H460 cell RNAs using standard RT- and PCR-kits and cloned into pcDNA3.1-derived vectors employing Gateway technology (Invitrogen). HEK 293T cells were transiently transfected with plasmids encoding HLA-A*01:01 and wildtype and mutated KRAS full-length and fragment cDNAs using Lipofectamine 2000 as per protocol (Invitrogen). Briefly, transfection was carried out on Multiscreen HTS 96-well plates as prepared for ELISpot analysis. Per well, 20,000 cells were transfected with 100ng HLA-plasmid, 300ng antigen-encoding plasmid and 0.5µl Lipofectamine transfection reagent. Twenty-four hours after transfection, recombinant 293T cells were used as target cells in IFN-γ ELISpot assays.

### IFN-γ ELISpot assays

HLA- and antigen-cDNA transfected 293T cells (20000/well), peptide pulsed (2µM) K562/HLA (50,000/well), NCI-H460, NCI-H460/HLA-A*01:01 cells (50,000/well), or tumor- and lung cell suspensions (20,000/well) were co-incubated with TCR-T cells (2,000-10,000 cTCR-positive cells/well) in IFN-γ antibody-coated Multiscreen HTS plates (Merck-Millipore, Darmstadt, Germany) overnight (16-20h). Positive control OKT3-antibody (from hybridoma, 400ng/ml) was co-coated in control-wells together with the anti-IFN-γ-antibody. After 20h, cells were discarded, developed according to manufacturer's instructions, supernatants were scanned and analyzed by ImmunoSpot Analyzer S5 Versa with ImmunoSpot software 7.0.15.1 (CTL Europe). For peptide-pulsing of K562/HLA-monoallelic cell lines, a Fast Track Peptide Library of candidate neoepitopes was purchased from JPT Peptide Technologies. Lyophilized peptide pools were reconstituted with DMSO and after dilution with RPMI (16µg/ml RPMI/5% DMSO) stored at -20°C. Pan-HLA antibody W6/32 (from hybridoma) was used to block pMHC-specific recognition of target cells by TCR-T cells.

### Cited references:

1. June, C.H., and Sadelain, M. (2018). N Engl J Med 379, 64-73. 10.1056/NEJMra1706169.
2. Cappell, K.M., and Kochenderfer, J.N. (2023). Nat Rev Clin Oncol 20, 359-371. 10.1038/s41571-023-00754-1.
3. John, M. (2023). OncoTargets and Therapy 16, 515-532. doi: 10.2147/OTT.S341179.
4. Schumacher, T.N., et al. (2019). Annu Rev Immunol 37, 173-200. 10.1146/annurev-immunol-042617-053402.
5. Lang, F., et al. (2022). Nat Rev Drug Discov. 10.1038/s41573-021-00387-y.
6. Hiltensperger, M., and Krackhardt, A.M. (2023). Current and future concepts for the generation and application of genetically engineered CAR-T and TCR-T cells. Front Immunol 14, 1121030. 10.3389/fimmu.2023.1121030.
7. Keam, S.J. (2024). Lifileucel: First Approval. Molecular Diagnosis & Therapy. 10.1007/s40291-024-00708-y.
8. Hong, D.S., et al. (2023). Nat Med. 10.1038/s41591-022-02128-z.
9. Johnson, L.A., et al. (2009). Blood 114, 535-546. 10.1182/blood-2009-03-211714.
10. Parkhurst, M.R., et al. (2011). Mol Ther 19, 620-626. 10.1038/mt.2010.272.
11. Morgan, R.A., et al. (2013). J Immunother 36, 133-151.
12. Yamamoto, T.N., et al. (2019). Nat Med. 10.1038/s41591-019-0596-y.
13. Tran, E., et al. (2016). New England J. Med. 375, 2255-2262.
14. Kim, S.P., et al. (2022). Cancer Immunol Res 10, 932-946. 10.1158/2326-6066.CIR-22-0040.
15. Leidner, R., et al. (2022). N Engl J Med 386, 2112-2119. 10.1056/NEJMoa2119662.
16. Seitz. V., et al. (2015). Nuc. Acids Re. 43:e135.
17. Roth T. L., et al. (2018). Nature 559: 405-409.
18. Kropp K. N. et al., (2023). Front. Immunol. 14. 10.3389.
19. Kropp K. N. et al., (2020) PLoS One 15, e0238875.10.1371.
20. EP3180433A1
21. EP3372683A1
22. WO 2017/025564 A1
23. Subbramanian et al. Nature Biotechnology, 22, 1429, (2004)

All scientific publications and patent documents cited in the present specification are incorporated by reference herein.

## Claims

1. An isolated T cell receptor comprising an alpha T cell receptor (TCRα) polypeptide comprising a complementarity determining region 3 alpha (CDR3α) sequence, and a beta T cell receptor (TCRβ) polypeptide comprising a complementarity determining region 3 beta (CDR3β) sequence; **characterized in that**
- said CDR3α sequence comprises, or consists of CALSEA(XXX)¹G(XX)²LX³F,
wherein (XXX)¹ is NQA, GSS, or KAA, (XX)² is TA, SA, or NK, and X³ is I or T (SEQ ID NO 022); and
said CDR3β sequence comprises, or consist of CASSR(XX)¹PP(XXXXX)²F, wherein (XX)¹ is KV, RW, RR or KL, and (XXXXX)² is VYGYT, VYGDT, TDTQY, or TYGYT (SEQ ID NO 023); or
- said CDR3α sequence comprises, or consists of a variant CDR3α sequence with one, or two amino acid substitutions compared to SEQ ID NO 022, and/or said CDR3β sequence comprises, or consists of a variant CDR3β sequence with one, or two amino acid substitutions compared to SEQ ID NO 023.

2. The isolated T cell receptor according to claim 1, said CDR3α sequence comprises, or consists of a variant CDR3α sequence with one, or two amino acid substitutions compared to SEQ ID NO 022, and/or said CDR3β sequence comprises, or consists of a variant CDR3β sequence with one, or two amino acid substitutions compared to SEQ ID NO 023, wherein the amino acid substitutions are selected according to the substitution rules:
- glycine (G) and alanine (A) are interchangeable; valine (V), leucine (L), and isoleucine (I) are interchangeable, A and V are interchangeable;
- tryptophan (W) and phenylalanine (F) are interchangeable, tyrosine (Y) and F are interchangeable;
- serine (S) and threonine (T) are interchangeable;
- aspartic acid (D) and glutamic acid (E) are interchangeable
- asparagine (N) and glutamine (Q) are interchangeable; N and S are interchangeable; N and D are interchangeable; E and Q are interchangeable;
- methionine (M) and Q are interchangeable;
- cysteine (C), A and S are interchangeable;
- proline (P), G and A are interchangeable;
- arginine (R) and lysine (K) are interchangeable.

3. The isolated T cell receptor according to claim 1 or 2, wherein
- the TCRα polypeptide comprises a sequence selected from the list consisting of SEQ ID NO 011, SEQ ID NO 013, SEQ ID NO 015, SEQ ID NO 017, and SEQ ID NO 019 or a sequence with ≥90%, ≥92%, ≥94%, ≥96%, ≥98%, or ≥99% sequence identity to SEQ ID NO 011, SEQ ID NO 013, SEQ ID NO 015, SEQ ID NO 017, and SEQ ID NO 019 and having substantially the same biological activity as SEQ ID NO 011, SEQ ID NO 013, SEQ ID NO 015, SEQ ID NO 017, or SEQ ID NO 019; and
- the TCRβ polypeptide comprises a sequence selected from the list consisting of SEQ ID NO 012, SEQ ID NO 014, SEQ ID NO 016, SEQ ID NO 018, and SEQ ID NO 020 or a sequence with ≥90%, ≥92%, ≥94%, ≥96%, ≥98%, or ≥99% sequence identity to SEQ ID NO 012, SEQ ID NO 014, SEQ ID NO 016, SEQ ID NO 018, and SEQ ID NO 020 and having substantially the same biological activity as SEQ ID NO 012, SEQ ID NO 014, SEQ ID NO 016, SEQ ID NO 018, and SEQ ID NO 020.

4. The isolated T cell receptor according to any of the preceding claims, wherein said CDR3α sequence and CDR3β sequence comprise, or consist of:
- SEQ ID NO 001, and SEQ ID NO 002;
- SEQ ID NO 003, and SEQ ID NO 004;
- SEQ ID NO 005 and SEQ ID NO 006;
- SEQ ID NO 007 and SEQ ID NO 008; or
- SEQ ID NO 009 and SEQ ID NO 010, respectively.

5. A nucleic acid encoding the T cell receptor according to any one of the claims 1 to 4.

6. An expression vector comprising the nucleic acid according to claim 5, under control of a promoter operable in a mammalian cell.

7. An engineered T cell comprising the T cell receptor as specified in any one of the claims 1 to 4, the nucleic acid according to claim 5, or the nucleic acid expression vector according to claim 6.

8. A pharmaceutical composition comprising the isolated T cell receptor according to any one of the claims 1 to 4, the nucleic acid according to claims 5, the nucleic acid expression vector according to claim 6, or the engineered cell according to claim 7.

9. The pharmaceutical composition according to claim 8, for use in treating a patient diagnosed with cancer, particularly wherein the cancer is lung cancer or gastrointestinal cancer.

10. The pharmaceutical composition for use according to claim 9, wherein the patient is **characterized by** an HLA-A*01:01 HLA allele.

11. The pharmaceutical composition for use according to claims 9 or 10, wherein the cancer is **characterized by** a HRAS, KRAS, or NRAS protein with a Q61H mutation, particularly wherein the cancer is **characterized by** a KRAS protein **characterized by** a Q61H mutation.

12. A method of determining a patient's likelihood of responding favourably to treatment with an engineered T cell, or pharmaceutical composition according to any one of the claims 7 to 11, wherein the method comprises the following steps:
- determining in a tumor nucleic acid sample obtained from said cancer patient if the patient's tumor is **characterized by** a HRAS, KRAS, or NRAS protein having a Q61H mutation;
- assigning the cancer patient a high likelihood of favorable response to treatment if the nucleic acid sample is **characterized by** a HRAS, KRAS, or NRAS protein having a Q61H mutation.

13. The method according to claim 12, further comprising a step of performing an HLA-typing on a sample obtained from the patient, wherein the patient is assigned a high likelihood of favorable response to treatment if the patient is **characterized by** an HLA-A*01:01 HLA allele.

14. The method according to claim 12 or 13, further comprising a step of
- determining in a nucleic acid sample obtained from said cancer patient whether a nucleic acid encoding the isolated T cell receptor according to any one of the claims 1 to 4 is present in the nucleic acid sample;
- assigning the cancer patient a high likelihood of favorable response to treatment if a nucleic acid encoding the isolated T cell receptor according to any one of the claims 1 to 4 is present in the nucleic acid sample.

15. A method of monitoring a cancer patient's T cell response to a tumor **characterized by** a Ras family protein having a Q61H mutation, or of monitoring the efficacy of a cancer patient's treatment, the patient having been administered an adoptive T cell therapy comprising an isolated T cell receptor according to any one of the claims 1 to 4,
the method comprising:
- determining an expression level of a nucleic acid encoding a CDR3α and/or CDR3β as specified by any one of the claims 1 to 4 present in a nucleic acid sample obtained from said cancer patient after commencement of treatment,
- comparing said expression level to a control expression level, particularly wherein the control expression level is obtained from a biological sample of the same tissue type lacking a TCR according the invention, or obtained from a baseline nucleic acid sample from said cancer patient before commencement of treatment.
